(19) Europäisches Patentamt / European Patent Office / Office européen des brevets

(11) **EP 4 036 931 B1**

(12) **EUROPEAN PATENT SPECIFICATION**

(45) Date of publication and mention
of the grant of the patent:
**23.07.2025   Bulletin 2025/30**

(21) Application number: **20867856.5**

(22) Date of filing: **25.09.2020**

(51) International Patent Classification (IPC):
*G16H 50/50* (2018.01)     *G16H 50/20* (2018.01)
*G16H 30/20* (2018.01)     *G06N 3/00* (2023.01)
*G06T 7/00* (2017.01)       *G06N 3/096* (2023.01)
*G06N 3/091* (2023.01)     *G06N 3/0895* (2023.01)
*G06N 3/088* (2023.01)     *G06N 3/09* (2023.01)
*G06N 3/094* (2023.01)     *G06N 3/0464* (2023.01)

(52) Cooperative Patent Classification (CPC):
**G06T 7/0012; G06N 3/0464; G06N 3/084;**
**G06N 3/088; G06N 3/0895; G06N 3/09;**
**G06N 3/091; G06N 3/094; G06N 3/096;**
G06N 3/045; G06T 2207/20081; G06T 2207/30096;
G06V 10/82; G06V 2201/03; G16H 50/20

(86) International application number:
**PCT/KR2020/013027**

(87) International publication number:
**WO 2021/060899 (01.04.2021 Gazette 2021/13)**

(54) **TRAINING METHOD FOR SPECIALIZING ARTIFICIAL INTELLIGENCE MODEL IN INSTITUTION FOR DEPLOYMENT, AND APPARATUS FOR TRAINING ARTIFICIAL INTELLIGENCE MODEL**

TRAININGSVERFAHREN ZUR SPEZIALISIERUNG EINES KÜNSTLICHEN INTELLIGENZMODELLS IN EINER INSTITUTION FÜR DEN EINSATZ UND VORRICHTUNG ZUM TRAINIEREN KÜNSTLICHER INTELLIGENZMODELLE

PROCÉDÉ D'APPRENTISSAGE POUR SPÉCIALISER UN MODÈLE D'INTELLIGENCE ARTIFICIELLE DANS UNE INSTITUTION POUR UN DÉPLOIEMENT ET APPAREIL POUR L'APPRENTISSAGE D'UN MODÈLE D'INTELLIGENCE ARTIFICIELLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.09.2019   KR 20190118545**
**24.09.2020   KR 20200124142**

(43) Date of publication of application:
**03.08.2022   Bulletin 2022/31**

(73) Proprietor: **Lunit Inc.**
**Seoul 06241 (KR)**

(72) Inventors:
• **YOO, Donggeun**
**Seoul 06241 (KR)**
• **PAEK, Seungwook**
**Seoul 06241 (KR)**
• **KIM, Minchul**
**Seoul 06241 (KR)**
• **PARK, Jongchan**
**Seoul 06241 (KR)**

(74) Representative: **Germain Maureau**
**12, rue Boileau**
**69006 Lyon (FR)**

(56) References cited:
JP-B2- 6 337 973     KR-A- 20170 034 258
KR-A- 20190 062 283     KR-A- 20190 106 861
US-A1- 2018 300 576     US-A1- 2018 314 943

- OZDEMIR FIRAT ET AL: "Extending pretrained segmentation networks with additional anatomical structures", INTERNATIONAL JOURNAL OF COMPUTER ASSISTED RADIOLOGY AND SURGERY, SPRINGER, DE, vol. 14, no. 7, 2 May 2019 (2019-05-02), pages 1187 - 1195, XP036808231, ISSN: 1861-6410, [retrieved on 20190502], DOI: 10.1007/S11548-019-01984-4
- LIN YANG ET AL: "Suggestive Annotation: A Deep Active Learning Framework for Biomedical Image Segmentation", ARXIV.ORG, CORNELL UNIVERSITY LIBRARY, 201 OLIN LIBRARY CORNELL UNIVERSITY ITHACA, NY 14853, 15 June 2017 (2017-06-15), XP080770025, DOI: 10.1007/978-3-319-66179-7_46
- BERKMAN SAHINER ET AL: "Deep learning in medical imaging and radiation therapy", MEDICAL PHYSICS., vol. 46, no. 1, 20 November 2018 (2018-11-20), US, pages e1 - e36, XP055698991, ISSN: 0094-2405, DOI: 10.1002/mp.13264
- ZHOU ZONGWEI ET AL: "Fine-Tuning Convolutional Neural Networks for Biomedical Image Analysis: Actively and Incrementally", 2017 IEEE CONFERENCE ON COMPUTER VISION AND PATTERN RECOGNITION (CVPR), IEEE COMPUTER SOCIETY, US, 21 July 2017 (2017-07-21), pages 4761 - 4772, XP033249832, ISSN: 1063-6919, [retrieved on 20171106], DOI: 10.1109/CVPR.2017.506

**Description**

**[Technical Field]**

The present disclosure relates to an artificial intelligence technology.

**[Background Art]**

Machine-learning technology, represented by deep-learning, provides results exceeding the performance of existing methods in analyzing various types of data such as images, voices, and texts. In addition, the machine learning technology is being applied to various fields due to the intrinsic scalability and flexibility of the technology, and various types of neural networks are being disclosed.

In this way, machine learning-based artificial intelligence (AI) technology is being actively adopted in the medical field. Previously, a computer aided detection (CAD) device performed a rule-based detection of a lesion or detected a lesion in a candidate area set in a medical image. However, recent AI-based medical image reading technology can analyze a whole medical image with AI algorithm and visually provide an abnormal lesion. An example of such work can be found in Ozdemir Firat et al. "Extending pretrained segmentation networks with additional anatomical structures".

Medical staff can receive information on the abnormal lesion included in the medical image from a diagnosis assistant device implemented with the AI-based medical image reading technology and then can diagnose with reference to the information.

Meanwhile, medical institutions are still using the same AI model despite differences in domains such as imaging equipment, imaging method, severity, and race. In this case, a difference between the data that each institution intends to analyze with an AI model and the training data of the AI model occurs. As a result, a problem that the performance of the AI model at the medical site is lower than expected is caused. Through fine-tuning with data of each institution, a pre-trained AI model can be optimized to the data of each institution. However, the AI model becomes to lose learned prior knowledge, which affects generalization performance. As a result, stable operation of the AI model cannot be guaranteed.

**[Disclosure]**

**[Technical Problem]**

The present disclosure provides a training method for specializing an artificial intelligence model in institutions for deployment and an apparatus for performing the same.

The present disclosure provides a method for collecting data of a deploying institution in order to train a pre-trained AI model. Specifically, a method for selecting data for training of the AI model among the data of the deploying institution and selecting data for which annotation is required is provided.

The present disclosure provides a method for training an AI model with data of a deploying institution while maintaining prior knowledge of the AI model.

**[Technical Solution]**

According to an embodiment, a method for operating a training apparatus operated by at least one processor is provided. The method includes extracting a dataset to be used for specialized training from data retained by a certain institution, selecting an annotation target for which annotation is required from the dataset by using a pre-trained artificial intelligence (AI) model, and performing supervised training of the pre-trained AI model by using data annotated with a label for the annotation target.

Selecting the annotation target includes selecting uncertain data to the pre-trained AI model as the annotation target by using a prediction result of the pre-trained AI model for at least some data in the dataset.

Selecting the annotation target may include selecting the annotation target based on an uncertainty score measured by using the prediction result of the pre-trained AI model.

The uncertainty score may be measured by using at least one of a confidence value of a score for each lesion predicted in the pre-trained AI model, entropy of a heatmap for each lesion predicted in the pre-trained AI model, and co-occurrence of lesions predicted in the pre-trained AI model.

Selecting the annotation target may include selecting, as the annotation data, data representing a distribution of the dataset in a feature space of the pre-trained AI model.

The method may further include annotating information extracted from a radiologist report on the annotation target, or supporting an annotation task by providing an annotator with a prediction result of the pre-trained AI model for the annotation target.

Extracting the dataset to be used for specialized training may include determining an amount of data to be used for specialized training, based on data retention amount and data characteristics of the certain institution.

Performing supervised training of the pre-trained AI model may include providing information for maintaining prior knowledge of the pre-trained AI model to the AI model under supervised training.

Performing supervised training of the pre-trained AI model may include calculating a distillation loss between the AI model under supervised training and a teacher model, and providing the distillation loss to the AI model under supervised training. Here, the teacher model is the same model as the pre-trained AI model.

The distillation loss may be a loss that makes the AI model under supervised training follow an intermediate feature and/or a final output of the teacher model.

According to another embodiment, a method for operating a training apparatus operated by at least one processor is provided. The method includes collecting a first dataset for pre-training, outputting a first AI model that has performed pre-training of at least one task using the first dataset, and outputting a second AI model that has performed specialized training using a second dataset collected from a certain institution while maintaining prior knowledge acquired in pre-training.

The first AI model may be trained with data that is pre-processed so as not to distinguish a domain of input data or may perform adversarial learning so as not to detect the domain of the input data from an extracted intermediate feature.

Outputting the second AI model may include calculating a distillation loss between the AI model under specialized training and a teacher model, and making the second AI model maintain the prior knowledge by providing the distillation loss to the AI model under specialized training. Here, the teacher model is the same model as the first pre-trained AI model.

Outputting the second AI model may include performing supervised training of the first AI model by using at least some of annotation data annotated with a label among the second dataset, and providing information for maintaining prior knowledge of the first AI model to the AI model under supervised training. The information for maintaining prior knowledge of the first AI model may be a distillation loss between the AI model under supervised training and a teacher model. The teacher model may be the same model as the first AI model.

The method may further include extracting the second dataset to be used for specialized training from the data retained by the certain institution, selecting an annotation target for which annotation is required from the second dataset by using the first AI model, and obtaining data annotated with a label for the annotation target.

Selecting the annotation target may include selecting, as the annotation target, uncertain data to the first AI model by using a prediction result of the first AI model for at least some data in the second dataset.

Selecting the annotation target may include selecting, as the annotation target, data representing a distribution of the second dataset in a feature space of the first AI model.

According to still another embodiment, a training apparatus is provided. The training apparatus includes a memory for storing instructions, and a processor for executing the instructions. The processor may extract a certain amount of medical institution data from a data repository of a medical institution, and perform specialized training of a pre-trained AI model by using the medical institution data while maintaining prior knowledge of the pre-trained AI model.

The processor may extract uncertain data to the pre-trained AI model from the medical institution data by using a prediction result of the pre-trained AI model for the medical institution data, select the uncertain data as an annotation target for which annotation is required, and perform supervised training of the pre-trained AI model using data annotated with a label for the annotation target. The processor may make the prior knowledge maintained by providing the AI model under supervised training with information for maintaining the prior knowledge.

The processor may select a certain number of representative data representing a distribution of the medical institution data, and select data for which a prediction of the pre-trained AI model is uncertain from the representative data. The uncertain data may be selected using at least one of a confidence value of a score for each lesion predicted in the pre-trained AI model, entropy of a heatmap for each lesion predicted in the pre-trained AI model, and co-occurrence of lesions predicted in the pre-trained AI model.

## [Advantageous Effects]

According to an embodiment, since various institutions can use an AI model specialized for domain characteristics of each institution, there is no need to worry about degradation of the AI model performance due to a difference in domains such as imaging equipment, imaging method, severity, and race.

According to an embodiment, the AI model may learn intrinsic data of each institution while maintaining prior knowledge for stable operation. Therefore, according to an embodiment, an AI model specialized for each institution can provide an analysis result reflecting intrinsic characteristics of each institution while providing generalization performance.

According to an embodiment, since data that is uncertain while representing data retained by each institution may be selected as an annotation target among the data retained by each institution, annotation only on the selected data may be required needless to annotate all data. Therefore, according to an embodiment, convenience in training may be increased, training time may be reduced, and training cost may be saved.

According to an embodiment, a provider providing an AI model to institutions may differentiate the AI model by changing an amount of collected data or an amount of data requiring annotation according to contract terms with each institution.

[Description of the Drawings]

FIG. 1 is a configuration diagram of a training apparatus according to an embodiment.

FIG. 2 is a diagram illustrating a specialized training method according to an embodiment.

FIG. 3 and FIG. 4 are diagrams illustrating a specialized training method according to another embodiment.

FIG. 5 is a diagram illustrating a specialized training method according to still another embodiment.

FIG. 6 is a diagram schematically illustrating specialized training according to an embodiment.

FIG. 7 is a flowchart showing a pre-training method according to an embodiment.

FIG. 8 is a flowchart showing a method for collecting data for specialized training according to an embodiment.

FIG. 9 is a flowchart showing a specialized training method according to an embodiment.

FIG. 10 is a configuration diagram of a computing device according to an embodiment.

[Mode for Invention]

In the following detailed description, only certain embodiments of the present invention have been shown and described, simply by way of illustration.

Throughout the specification, when a part is referred to "include" a certain element, it means that it may further include other elements rather than exclude other elements, unless specifically indicates otherwise.

In the description, "transmission or provision" may include direct transmission or provision, as well as indirect transmission or provision through other devices or by way of bypass.

In the description, expressions described in the singular in this specification may be interpreted as the singular or plural unless an explicit expression such as "one" or "single" is used.

In the flowchart described with reference to drawings in this description, the operation order may be changed, several operations may be merged, certain operations may be divided, and specific operations may not be performed.

In the description, the terms such as "... unit", "... er/or", "... module", and the like refer to units that process at least one function or operation, which may be implemented with a hardware, a software or a combination thereof.

In the description, an apparatus is configured and connected so that at least one processor can perform operations of the present disclosure by executing instructions. The computer program includes instructions that are described for a processor to execute the operations of the present disclosure, and may be stored in a non-transitory computer readable storage medium. The computer program may be downloaded via a network or sold as a product.

An artificial intelligence model (AI model) of the present disclosure is a machine learning model that learns at least one task, and may be implemented as a computer program executed by a processor. The task that the AI model learns may refer to a task to be solved through machine learning or a task to be executed through machine learning. For example, when it is assumed that recognition, classification, and prediction from a medical image are executed, each of the recognition, classification, and prediction may correspond to individual tasks.

The AI model of the present disclosure may be configured with various neural networkbased machine learning models to fit for input data, task types, learning methods, and the like. For example, when an AI model receives a medical image as an input, a convolutional neural network (CNN) model may be used.

The AI model of the present disclosure may receive various types of data. In the description, an AI model that uses a medical image as training data and analysis data may be described as an example, and the AI model that receives the medical image and performs at least one task may be configured to have various structures.

The present disclosure can be applied to medical images of various regions taken with various modalities. For example, the modality of the medical image may be various, such as X-ray, magnetic resonance imaging (MRI), ultrasound, computed tomography (CT), mammography (MMG), and digital breast tomosynthesis (DBT).

In the description, the term data can be used interchangeably with the term dataset.

In the description, "deploying institution" means an agent that deploys an AI model or a device including the AI model (e.g., diagnosis assistant device), or a place (a facility) where the AI model or the device including the AI model is deployed. For example, the deploying institution may include a hospital, a medical check-up center, a company, a school, a public institution, and the like. The "deploying institution" may be briefly referred to as "institution", or may be referred to as "target institution", "medical institution", "target hospital", "using place", and the like.

In the description, deploying institution data is data retained by the deploying institution, and may be, for example, a medical image taken by an imaging device equipped in the deploying institution or a medical image for which the deploying institution receives a request from the outside. The deploying institution data may be, for example, medical images stored in a picture archiving and communication system (PACS) of a hospital.

In the description, "specialization" refers to a process or operation that makes a pre-trained AI model output good performance even for the deploying institution data (e.g., a medical image), and may include an operation of optimizing the pre-trained AI model to the deploying institution, an operation of fine-tuning the pre-trained AI model for the deploying institution, an operation of customizing the pre-trained AI model for the deploying institution, and the like. Here, "good performance" may mean a case in which a result output from the AI model for the deploying institution data shows a performance similar to or better than a "reference performance". The "reference performance" may be set various and may be, for example, a performance indicator of a pre-trained AI model evaluated with validation data.

In the description, training for specializing a pre-trained AI model for a deploying institution may be referred to as "specialized training", and may also be referred to as "additional training".

In the description, a pre-trained AI model may be an AI model that has completed learning so that the AI model can be used by the deploying institution without additional specialized training.

In the description, data for pre-training of an AI model may be referred to as pre-training data, and additionally may be referred to as in-house data of a company developing the AI model, basic data, source data, and the like.

In the description, the deploying institution data may be referred to as target-hospital data, target data, and the like.

In general, an AI model learns a task using training data, and finishes learning when a result evaluated with validation data reaches a predetermined performance. Though the training data and the validation data may be data obtained under various conditions and environments, it is difficult for them to reflect all conditions and all environments. Therefore, upon inputting data actually taken in a hospital to an AI model, even an AI model having completed training outputs a result that falls short of validation performance.

This problem may be caused by a difference in a domain, being an environment or condition where the data is collected/generated. The domain difference may be caused by, for example, diversity in imaging equipment, imaging method, severity, race, and the like.

For example, when equipment that imaged the pre-training data is different from equipment that imaged the data at the deploying institution, texture of the images may be different. When an imaging method of the pre-training data is different from an imaging method utilized by the deploying institution, information included in the images may be different, and performance may be deteriorated due to the difference in the images. For example, different imaging methods such as anterior posterior (AP) imaging or posterior anterior (PA) imaging may be used, and imaging may be performed so that a hand is seen or not.

A severity distribution of the pre-training data may be different from that of the institution data. For example, though the pre-training data had been collected from a hospital having a large number of patients with relatively high severity, the deploying institution may be a hospital having a large number of patients with relatively low severity.

A racial distribution of the pre-training data may be different from that of the institution data. For example, though the pre-training data had been collected in a hospital used by Asians, the deploying institution may be a hospital that patients of different racial distribution use.

Thus, when each deploying institution analyzes data with an AI model having completed training, a problem that the AI model shows a lower performance than expected due to such domain differences should be solved. Here, when the pre-trained AI model is fine-tuned simply by using the institution data, the AI model becomes to forget prior knowledge obtained through pre-training, thereby affecting generalization performance. As a result, stable operation of the AI model cannot be guaranteed.

Therefore, hereinafter, a method for training an AI model utilizing data of deploying institution while maintaining prior knowledge of the AI model will be described in detail. And, hereinafter, a method for selecting data for training an AI model from deploying institution data and selecting data for which annotation is required will be described in detail.

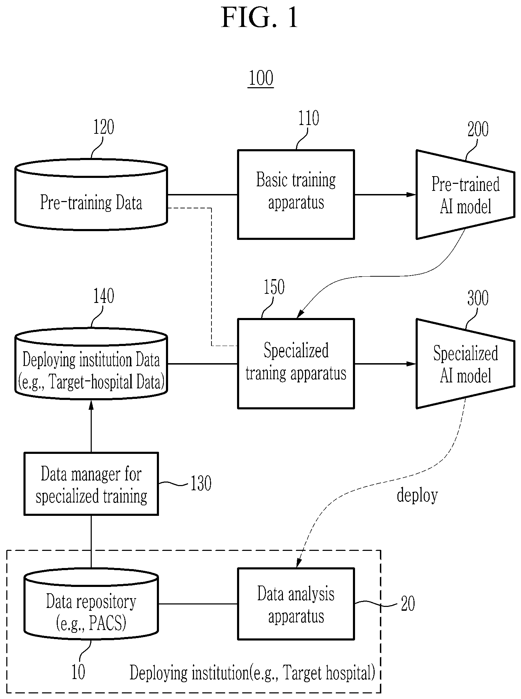

FIG. 1 is a configuration diagram of a training apparatus according to an embodiment.

Referring to FIG. 1, the training apparatus 100 may include a basic training apparatus 110, a data manager for specialized training (briefly, referred to as a "data manager") 130, and a specialized training apparatus 150 that perform specialized training of a pre-trained AI model 200 with deploying institution data. For convenience of description, a deploying institution may be referred to as a target hospital, and the deploying institution data may be referred to as target-hospital data.

The basic training apparatus 110 is connected with database 120 in which pre-training data is stored, and outputs an AI model 200 that has learned at least one task by using the pre-training data of the database 120. The AI model 200 may be referred to as a basic AI model, a pre-trained AI model, a general AI model, and the like.

The data manager 130 may select target-hospital data for specialized training from a data repository 10 of a deploying institution, and store the target-hospital data at least some of which is annotated, in database 140.

The specialized training apparatus 150 is connected with the database 140 in which the target-hospital data is stored, performs specialized training of the pre-trained AI model 200 using the target-hospital data of the database 140, and then outputs an AI model 300 for the deploying institution. The specialized training apparatus 150 may use the pre-training data of the database 120 for specialized training. The AI model 300 that has completed specialized training in the specialized training apparatus 150 may be provided to a corresponding deploying institution. The specially trained AI model (specialized AI model) 300 may be mounted on, for example, a data analysis apparatus 20 (e.g., an image reading apparatus) of the corresponding institution.

The data manager 130 or the specialized training apparatus 150 may be centrally located, such as in a cloud server, may be connected with a plurality of deploying institutions, may perform specialized training requested by the plurality of deploying institutions, and then may provide the AI model to the corresponding institutions. Alternatively, the data manager 130 or the specialized training apparatus 150 may be arranged in each institution, thereby performing specialized training individually.

Though the basic training apparatus 110, the data manager 130, and the specialized training apparatus 150 are named separately for the sake of explanation, they may be a computing device operated by at least one processor. Here, the basic training apparatus 110, the data manager 130, and the specialized training apparatus 150 may be implemented on one computing device or with separate computing devices in a distributed manner. When implemented with separate computing devices in a distributed manner, the basic training apparatus 110, the data manager 130, and the specialized training apparatus 150 may communicate with each other via a communication interface.

On the other hand, the basic training apparatus 110, the data manager 130, and the specialized training apparatus 150 may be implemented with a machine learning model required for training the AI model. In the description, the AI model 200 and the AI model 300 may be referred to as target models to be built through machine learning.

The basic training apparatus 110 outputs the AI model 200 that has learned at least one task using the pre-training data. Here, the pre-training data may be composed of data obtained by various institutions and/or data obtained from various equipment. In addition, the pre-training data may include data obtained with various imaging methods. As described above, when collecting as much data as possible as the pre-training data, there may be an inevitable domain difference. Therefore, it is necessary to reduce the domain difference in the input data through an operation of domain generalization.

The basic training apparatus 110 may pre-process training data in order to reduce the domain difference in the input data, and then train the AI model 200 using the pre-processed training data.

For example, images acquired in different domains have differences in the texture, and the like. The basic training apparatus 110 may remove a unique image feature appearing in a domain so that the AI model 200 cannot distinguish from which institution or with which equipment the input image is obtained. The basic training apparatus 110 may perform pre-processing of removing domain features of images obtained from different domains, through image-to-image translation. For example, the basic training apparatus 110 may use a generative adversarial network (GAN) as an image-to-image translation model, and may perform image-to-image translation using a discriminator and an adversarial loss so that the discriminator cannot detect the domain of the images.

In addition to domain generalization at the image level, the basic training apparatus 110 may perform domain generalization at intermediate features extracted from an intermediate layer of the AI model 200. The basic training apparatus 110 may train the AI model through adversarial training so that the discriminator cannot discern the domain from the intermediate features of the input image.

The data manager 130 extracts a certain amount of the target-hospital data for performing specialized training of the pre-trained AI model 200, from the data repository 10 of the deploying institution. The data manager 130 may determine at least some of the imported target-hospital data as training data for specialized training, and provide the training data to the specialized training apparatus 150. At this time, the data manager 130 may determine at least some of the training data as an annotation target, and provide specialized training apparatus 150 with the training data at least some of which is annotated. The training data may include abnormal data and normal data.

The data manager 130 may determine validation data for evaluating the specialized AI model 300 from the target-hospital data, and provide the validation data to the specialized training apparatus 150. The validation data may be collected so as not to overlap with the training data. For example, as for the validation data, N cases of abnormal data may be collected for each of C lesions and N*C cases of normal data may be collected.

The data repository 10 of the deploying institution may be, for example, medical images stored in a picture archiving and communication system (PACS) of a target hospital. The data manager 130 may be allowed to access the data repositories of the deploying institutions and directly extract the data. Otherwise, the data manager 130 may acquire necessary information from an intermediate device connected with the data repositories of the deploying institutions.

The data manager 130 may determine the amount of data to be used for specialized training, in the data repository of the deploying institution. The data manager 130 may determine the amount of data to be used for training in consideration of data retention amount and data characteristics of the deploying institution. Data characteristics may include severity data rates, age distribution, gender distribution, racial distribution, and the like. For example, when the deploying institution is a university hospital or a medical check-up center, a proportion of abnormal data to the entire data may be different. Therefore, an amount of data imported by each deploying institution may vary in consideration of the data characteristics of each institution.

The data manager 130 may determine an amount of annotated data required for training. Since performing annotation takes time and cost, the amount of annotation data may be determined according to a request from the deploying institution or a contract therewith. In order to reduce costs, the specialized training can be performed without annotating the target-hospital data. Alternatively, in consideration of the performance of the specialized AI model 300, the specialized training may be performed using data at least some of which is annotated among the target-hospital data. In this case, the amount of annotation data may be determined according to the willingness-to-pay of the institution.

The annotation can be performed with an image-level label or a pixel-level label. For example, an image-level label on which whether there is a malignant lesion or not is annotated or a pixel-level label where a lesion is indicated as a contour may be possible. Types and levels of annotations may be variously determined depending on the level of labels that can be provided by the deploying institution or annotation cost.

The annotation method may be various. The label may be manually annotated on the data by a person, or the label extracted from a report written by a doctor who specializes in image reading (a radiologist, etc.) can be automatically annotated on the corresponding image.

The data manager 130 may select data for which the annotation is required (briefly referred to as an "annotation target"), among the target-hospital data. At this time, the data manager 130 may select data uncertain to the pre-trained AI model 200 as the annotation target, or select data representing a distribution of the target-hospital data as the annotation target. Otherwise, the data manager 130 may select, as the annotation target, data that is uncertain (unclear) to the pre-trained AI model 200 while representing the distribution of the target-hospital data. If there is data for which a radiologist report exists, the data manager 130 may select the annotation target from such data.

A method for the data manager 130 to select the annotation target may be a method according to an example or a combination of examples described below.

For example, the data manager 130 may select the annotation target by measuring uncertainty and/or diversity of the extracted target-hospital data. The data manager 130 may select data for which prediction of the pre-trained AI model is uncertain, among a predetermined number of representative data representing the target-hospital data.

The data manager 130 may measure uncertainty for at least some data of the target-hospital data, and then select the annotation target. The data manager 130 defines an uncertainty score using a prediction value of the pre-trained AI model 200, and may select, as the annotation target, data having an uncertainty score equal to or greater than a reference or top k data having the largest uncertainty score.

The uncertainty score may be defined, for example, as follows.

According to an embodiment, uncertainty may be measured by using a confidence value of a score predicted for each lesion in the pre-trained AI model 200. The uncertainty score for each lesion predicted for the data in the pre-trained AI model 200 may be defined as shown in Equation 1, and the uncertainty score of the data may be set as a maximum value or an average value of the uncertainty scores of the lesions. Here, the lesion score is a probability value between 0 and 1, and the uncertainty score is defined so that the uncertainty increases as the lesion score reaches an intermediate value not unquestionably positive or negative.

$$\underline{\text{Equation 1}}$$

$$\text{Uncertainty score of lesion} = 1 - |\text{lesion score} - 0.5|$$

According to another embodiment, uncertainty may be measured by using entropy of a heatmap for each lesion predicted in the pre-trained AI model 200. An uncertainty score for each lesion may be defined as entropy measured by regarding a two-dimensional heat map for each lesion as one one-dimensional vector. The uncertainty score of data may be set as the maximum value or the average value of the uncertainty scores of the lesions. Here, the entropy has a higher value as values placed in a vector are similar to each other, which means that the pre-trained AI model 200 does not clearly detect lesions. As a result, the higher the entropy is, the higher the uncertainty score gets.

According to yet another embodiment, uncertainty may be measured according to cooccurrence of lesions. As the scores of the lesions predicted in the pre-trained AI model 200 get similar, the AI model has more difficulty in predicting a lesion from the data. As a result, the uncertainty score gets high. The data manager 130 may make a vector of length C by collecting the scores of the C lesions, and then acquire the uncertainty score of the data through measuring the entropy of the vector. As described above, the more similar the values placed in the vector become, the higher the uncertainty score becomes.

Alternatively, the data manager 130 may measure the uncertainty score by using a difference between the top two lesion scores among the scores of C lesions, as shown in Equation 2. That is, the smaller the difference between the top two lesion scores gets, the AI model 200 has more difficulty in distinguishing lesions certainly. As a result, the uncertainty score gets high.

Equation 2

$$\text{Uncertainty score of data} = 1 - |\text{largest lesion score} - \text{second largest lesion score}|$$

A method for calculating an uncertainty score based on the co-occurrence of lesions will be described using an example. Referring to Table 1, it is assumed that the AI model 200 outputs a score (probability) for data 1 and data 2 that they fall within 5 lesions or classes. Comparing results predicted for data 1 and data 2, a difference in five values (0.8, 0.7, 0.2, 0.4, 0.3) constituting a vector of data 2 is more similar than that of data 1. Thus, the uncertainty score of data 2 may be measured higher than that of data 1. Meanwhile, a difference in lesion scores of the top two (a=0.8, b=0.7) shown in data 2 is 0.1, and a difference in lesion scores of the top two (a=0.8, b=0.2) shown in data 1 is 0.6. As a result, the uncertainty score of data 2 may be measured higher than that of data 1. Accordingly, the data manager 130 may select data 2 as an annotation target, and then perform an annotation on data 2 or request annotation thereon.

Table 1

| Lesion or class | a | b | c | d | e |
|---|---|---|---|---|---|
| Predicted score for data 1 | 0.8 | 0.2 | 0.1 | 0.2 | 0.2 |
| Predicted score for data 2 | 0.8 | 0.7 | 0.2 | 0.4 | 0.3 |

The data manager 130 may select, as the annotation target, data representing a distribution of the target-hospital data in a feature space of the pre-trained AI model 200. The data manager 130 may measure diversity, select k representative data, and determine the selected data as the annotation target. A method for selecting k representative data may be various. For example, k data may be randomly sampled among the target-hospital data. After performing k-means clustering for the target-hospital data, k data closest to each cluster centroid may be selected. By using k-center greedy algorithm, k data that can cover the entire distribution of the target-hospital data with a delta ($\delta$) radius may be selected. Alternatively, k data that can cover the entire distribution of target-hospital data with the delta ($\delta$) radius may be selected by using robust k-center algorithm.

If there is data for which a radiologist report exists, the data manager 130 may select the annotation target among such data. In this case, the data manager 130 may select the annotation target in consideration of a lesion for specialized training and a positive/negative proportion. In order to extract, from the report, information including whether there is a lesion, the data manager 130 may use a separate language processing model. For example, a natural language processing (NLP) model, a deep language model, and the like may be used as the language processing model.

The data manager 130 may select the annotation target by using a prediction value of the pre-trained AI model 200 for the data for which a radiologist report exists. Data for which information of the report does not match the prediction of the AI model therefor may be selected as the annotation target.

Annotation on the annotation target selected by the data manager 130 may be variously executed.

For example, the data manager 130 may provide the annotation target to an annotation device, and may receive data annotated with a label from the annotation device. An annotator may write a label on the data in the annotation device or the annotation device may automatically or semi-automatically write the label on the data.

When a radiologist report exists, the data manager 130 may annotate the corresponding data with information extracted from the radiologist report.

For the sake of the annotator, the data manager 130 may provide the prediction result of the AI model 200 for the annotation target and operate to make an annotation task be executed semi-automatically. A score for each lesion, a contour, an abnormality score of data, and the like may be provided to the annotator as the prediction result of the AI model 200, and the annotator may perform an accurate and quick annotation with reference to the prediction result.

Meanwhile, when the specialized training is performed without annotating the target-hospital data for cost reduction, the data manager 130 may provide all the imported target-hospital data as the training data. Alternatively, the data manager 130 may select some data as the training data among the imported target-hospital data, by using metrics such as a score for each lesion, an uncertainty score for each lesion, and an abnormality score. Metrics such as the score for each lesion, the uncertainty score for each lesion, and the abnormality score may be extracted from the pre-trained AI model. For example, the top N% having a high prediction score for each lesion may be selected as the training data. Alternatively, a certain proportion of the training data may be selected for each score range. For example, a predetermined proportion of the data may be selected as the training data in each of multiple ranges obtained by dividing [0,1].

The specialized training apparatus 150 trains the pre-trained AI model 200 by using the training data received from the data manager 130 and generates an AI model 300 for a deploying institution. At this time, an amount of the target-hospital data or the annotated data may not be much enough for training. In addition, in the process that the pre-trained AI model performs additional learning using new data not being enough, a trouble of catastrophic forgetting that the pre-trained AI model becomes to forget the previously learned prior knowledge may be caused. Accordingly, the specialized training apparatus 150 uses a training method that enables the AI model 200 to remember the prior knowledge. Hereinafter, the method will be described in detail. The training method for remembering the prior knowledge may be referred to as learning without forgetting prior knowledge.

The specialized training apparatus 150 may determine a specialized training method in consideration of an amount of training data, an amount of annotated data, characteristics of the training data, and the like.

The training data for the specialized training may include data, at least some of which is annotated. When the training data includes annotated data, supervised training may be used. When the training data does not include the annotated data, semi-supervised learning or unsupervised domain adaptation may be used.

The specialized training apparatus 150 may define a loss that prevents the prior knowledge of the pre-trained AI model 200 from being changed, and perform specialized training with the defined loss. For example, supervised loss may be used for the annotated target-hospital data. For unannotated target-hospital data, unsupervised loss may be used. In addition, a loss that makes predictions of some data used for pre-training of the AI model 200 unchanged may be used. The specialized training apparatus 150 may use at least some of the losses defined for the specialized training to perform training of the AI model utilizing the target-hospital data.

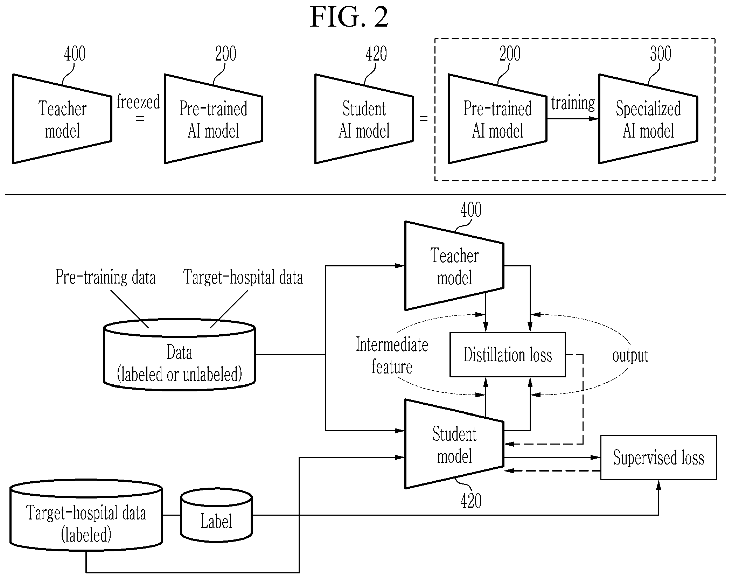

FIG. 2 is a diagram illustrating a specialized training method according to an embodiment.

Referring to FIG. 2, the specialized training apparatus 150 generates an AI model 300 specialized for target-hospital data while remembering prior knowledge acquired through pre-training. For this, the specialized training apparatus 150 may utilize knowledge distillation that a student model learns while emulating a teacher model.

The deploying institution may be a targeted hospital, and data of the deploying institution data may be target-hospital data.

For knowledge distillation-based training, a teacher model 400 is a pre-trained AI model 200, and is frozen as the pre-trained AI model 200 for knowledge distillation-based learning. A student model 420 prior to learning is a pre-trained AI model 200. The pre-trained AI model 200 may become a specialized AI model 300 after performing specialized training without forgetting prior knowledge retained therein. In general, knowledge distillation is used to generate a small student model by emulating a large teacher model. On the contrary, an initial student model 420 in the present disclosure may be the same AI model 200 as the teacher model 400, prior to training. And then the initial student model 420 may finally turn to the specialized AI model 300 through specialized training utilizing the target-hospital data. That is, the student model 420 of the present disclosure receives help from the teacher model 400 in order to maintain the prior knowledge that the student model 420 originally has while performing specialized training with the target-hospital data. In the description, it is explained that the initial model of the student model 420 is the pre-trained AI model 200. Even though the initial model of the student model 420 is not necessarily the pre-trained AI model 200, any model that can learn the prior knowledge transferred from the teacher model 400 is available.

The specialized training apparatus 150 arranges the pre-trained AI model 200 as the teacher model 400, and arranges the pre-trained AI model 200 as the student model 420, and then progresses prior knowledge maintenance training and specialized training utilizing the target-hospital data for the student model 420. Through progressing prior knowledge maintenance training while performing specialized training of the student model 420, the student model 420 may be trained so as not to forget the prior knowledge, due to specialized training. The specialized training apparatus 150 may perform weighted summation of a loss calculated during prior knowledge maintenance training (distillation loss) and a loss calculated during specialized training (supervised loss), and then may train the student model 420 by applying backpropagation of weighted-summed loss to the student model 420. The weights of the distillation loss and the supervised loss may be variously determined.

For prior knowledge maintenance training, pre-training data used for pre-training of the AI model 200 or the target-hospital data may be used. Data for prior knowledge maintenance training may be determined according to a data range accessible by the specialized training apparatus 150. At this time, the data for prior knowledge maintenance training may not have a label. The data used for prior knowledge maintenance training may be selected from the pre-training data or the target-hospital data, by using metrics such as a score for each lesion, an uncertainty score for each lesion, and an abnormality score. For example, the data of top N% having a high score for each lesion may be selected as the training data. Alternatively, the training data of a certain proportion may be selected for each score range, and for example, data of a predetermined proportion in each of multiple ranges obtained by dividing [0,1] may be selected as the training data.

The prior knowledge maintenance training may be performed as follows.

The specialized training apparatus 150 inputs the same data to the teacher model 400 and the student model 420. The specialized training apparatus 150 calculates a distillation loss that makes intermediate features and/or outputs obtainable from the teacher model 400 and the student model 420 similar to each other, and then provides the student model 420 with the calculated distillation loss. As a result, the student model can be trained so as to output a similar value to the teacher model 400. Through the above processes, the student model 420 can remember prior knowledge. L1/L2 loss between Gram matrices of two intermediate features, cosine similarity or L1/L2 loss may be used as the distillation loss using the intermediate feature. Cross-entropy between predicted values of two models may be used as the distillation loss using a final output.

For specialized training, label-annotated target-hospital data can be used. Meanwhile, for specialized training, pre-training data used for pre-training of the AI model 200 may be used together.

The specialized training apparatus 150 may perform supervised training of the student model 420 using the annotated data. The specialized training apparatus 150 may calculate a supervised loss that is a difference between a predicted value for the input data by the student model 420 and a label of the input data, and perform supervised training of the student model 420 by providing the calculated supervised loss to the student model 420. The cross-entropy, binary cross-entropy, and the like may be used as the supervised loss.

The specialized training apparatus 150 may validate the student model 420 that has completed prior knowledge maintenance training and specialized training by using validation data extracted from the target-hospital data. The validation data may be different from data used for training.

When performance validation is satisfied, the specialized training apparatus 150 may terminate training of the student model 420 and provide the student model 420 to the deploying institution.

If the performance validation is unsatisfied, the specialized training apparatus 150 may iterate training of the student model 420 until the performance validation is satisfied. To this end, the specialized training apparatus 150 may request the data manager 130 to reselect an annotation target among the target-hospital data and iterate the training using the reselected data.

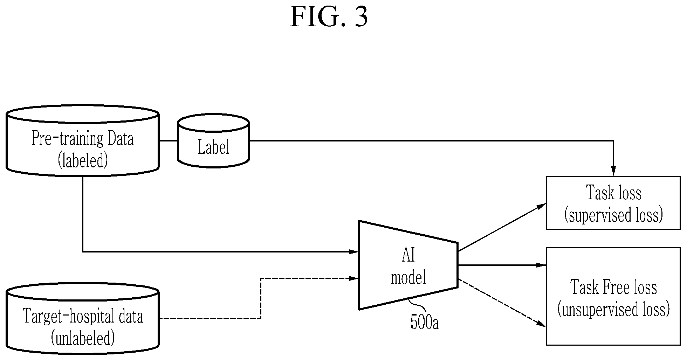

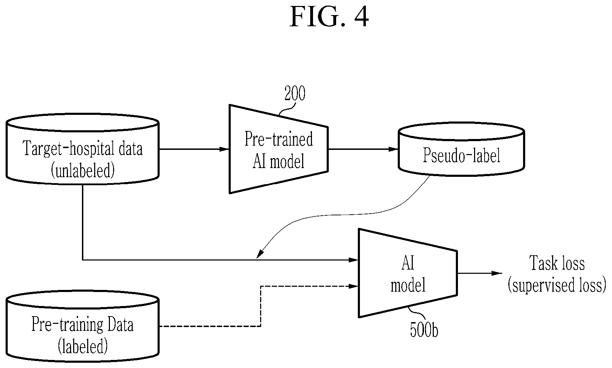

FIG. 3 and FIG. 4 are diagrams illustrating a specialized training method according to another embodiment.

Methods that a specialized training apparatus 150 trains AI models 500a and 500b, being targets of training, using unannotated target-hospital data will be described with reference to FIG. 3 and FIG. 4.

First, referring to FIG. 3, the specialized training apparatus 150 may perform training by which the target-hospital data is adapted to pre-training data with a different domain through unsupervised domain adaptation. Here, it is assumed that the pre-training data is data annotated with a label for a task and the target-hospital data is data unannotated with a label. The initial model of the AI model 500a may be a pre-trained AI model 200. Alternatively, even though not being the pre-trained AI model 200, the initial model of the AI model 500a may be a model capable of learning the pre-training data and the target-hospital data.

The AI model 500a learns an arbitrary task (task free) possible without a label while learning a task using pre-training data with a label. Further, the AI model 500a learns an arbitrary task possible without a label by using the target-hospital data that is not annotated with a label. The AI model 500a may learn a task free loss/unsupervised loss while learning a task loss/supervised loss.

Learning possible without a label is, for example, domain classification learning, domain adversarial learning, self-supervised learning, and the like. The domain classification learning is learning for identifying from which institution input data is acquired. The domain adversarial learning is learning for generating a feature that makes it not possible to identify from which institution the input data is acquired. The self-supervised learning is learning through making a label by itself with data retained by the model, like rotation prediction.

The specialized training apparatus 150 may validate the AI model 500a by using the validation data extracted from the target-hospital data. The validation data may be data different from the data used for training. When the performance validation is satisfied, training of the AI model 500a may be terminated and then the deploying institution may be provided with the AI model 500a.

Referring to FIG. 4, a specialized training apparatus 150 may acquire a pseudo-label of target-hospital data by using a result predicted from the target-hospital data in a pre-trained AI model 200. Here, it is assumed that the pre-training data is data annotated with a label for a task and the target-hospital data is data not annotated with a label.

The specialized training apparatus 150 may train the AI model 500b by using the target-hospital data annotated with the pseudo-label and the pre-training data annotated with a label. An initial model of the AI model 500b may be the pre-trained AI model 200. Alternatively, even though not being the pre-trained AI model 200, the initial model of the AI model 500b may be a model capable of learning the pre-training data and the target-hospital data.

The specialized training apparatus 150 may validate the AI model 500b by using validation data extracted from the target-hospital data. The validation data may be data different from the data used for training. When the performance validation is satisfied, training of the AI model 500b may be terminated and may be provided to a deploying institution.

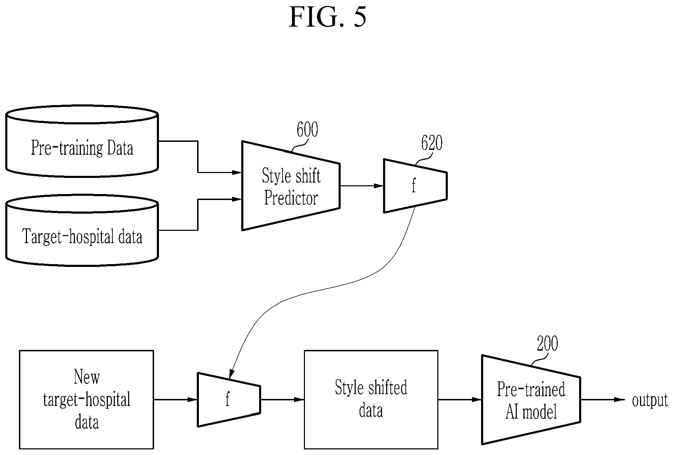

FIG. 5 is a diagram illustrating a specialized training method according to still another embodiment.

Referring to FIG. 5, for training using unannotated target-hospital data, a specialized training apparatus 150 may train a style shift predictor 600.

The style shift predictor 600 may receive target-hospital data and pre-training data, and may perform learning for finding a style shift function (f) 620 that reduces a difference between a style distribution of the target-hospital data and that of the pre-training data. In this case, the style shift function (f) may be an invertible function that does not convert an amount of information of an image.

The style distribution of data may be defined in various ways. For example, when brightness of an image is defined as a style, the specialized training apparatus 150 can find a brightness conversion function that adjusts a brightness average of images used for pre-training and that of images received from the deploying institution, via the style shift predictor 600. Alternatively, the style may be defined as an average and a variance of features included in the data. The style may be defined as various scalars or vectors that can represent image styles.

The specialized training apparatus 150 may convert new target-hospital data with the style shift function (f) and validate the style shift function (f) by inputting the style-converted data into the pre-trained AI model 200.

Then, the new target-hospital data generated by the deploying institution is converted with a style conversion function (f) and the converted data is input to the pre-trained AI model 200.

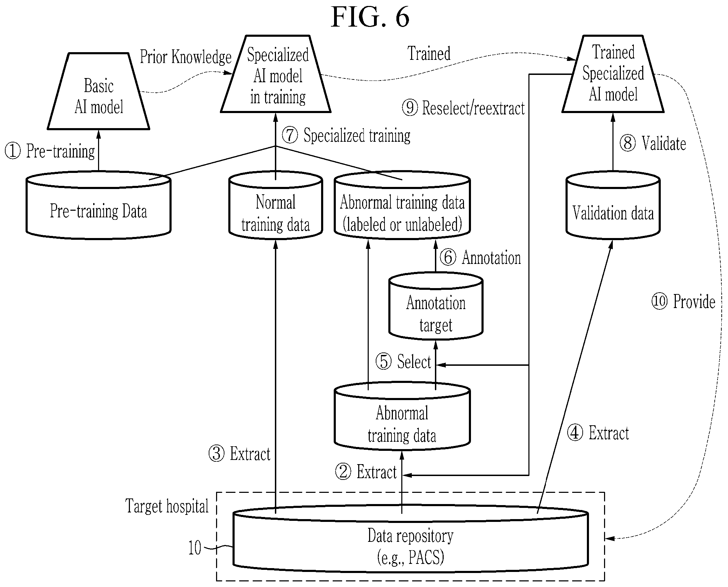

FIG. 6 is a diagram schematically illustrating specialized training according to an embodiment.

Referring to FIG. 6, a basic AI model 200 is pre-trained by pre-training data (①). The AI model 200 may receive pre-processed pre-training data in order to reduce a domain difference in input data. In the pre-training data, domain features of images acquired from different domains may be removed through image-to-image translation. Alternatively, the AI model 200 may perform learning for removing domain features of intermediate features. Through adversarial learning, domain generalization for reducing the domain difference in the pre-training data may be performed.

From a data repository 10 of the deploying institution, abnormal training data, normal training data, and validation data are extracted (②, ③, ④). A training apparatus 100 may determine an amount of data to be used for specialized training in consideration of data retention amount of the data repository 10 and data features.

An annotation target requiring annotation is selected from the abnormal training data (⑤). Data uncertain to the pre-trained AI model 200 or data representing a distribution of the target-hospital data may be selected as the annotation target. Alternatively, data that is uncertain to the pre-trained AI 200 while representing the distribution of the target-hospital data may be selected as the annotation target. The annotation target may be selected based on uncertainty and/or diversity of the target-hospital data. The uncertainty may be measured by using a confidence value of a score for each lesion predicted in the pre-trained AI model 200, measured by using entropy of a heatmap for each lesion predicted in the pre-trained AI model 200, or measured by considering co-occurrence of lesions. For the diversity representing the distribution of the target-hospital data, the annotation target may be randomly sampled or k representative data may be selected as the annotation target. The annotation target may be selected among data for which a radiologist report exists.

A label annotated on the annotation target is provided (⑥). The training apparatus 100 may provide an annotation device with the annotation target, and may receive data annotated with a label from the annotation device. An annotator may label data in the annotation device, or the annotation device may automatically/semi-automatically label the data. The training apparatus 100 may annotate information extracted from the radiologist report as a label of the annotation target. For the sake of the annotator, the training apparatus 100 may provide a prediction result of the AI model 200 for the annotation target, and operate so that an annotation task is semi-automatically executed.

The AI model 300 performs specialized training with the training data (⑦). The AI model 300 performs specialized training using the target-hospital data while remembering prior knowledge. For this, as described above with reference to FIG. 2, the AI model 300 may perform training (prior knowledge maintenance training) that makes prior knowledge retained by the basic AI model 200 not forgotten while learning a distillation loss provided by the basic AI model 200. The AI model 300 may perform specialized training using the basic AI model 200 as an initial model.

The specialized AI model 300 is validated with validation data (⑧).

When the performance validation falls short of a reference, the abnormal training data or the annotation target may be reselected from the data repository 10 of the deploying institution (⑨). Using the reselected training data or the reselected annotation target, the AI model 300 performs specialized learning again.

When the performance validation is satisfied, training of the AI model 300 is terminated and the AI model 300 is provided to the deploying institution (⑩).

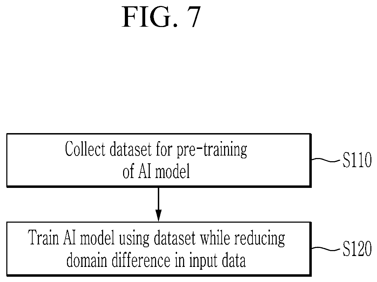

FIG. 7 is a flowchart showing a pre-training method according to an embodiment.

Referring to FIG. 7, a training apparatus 100 collects a dataset for pre-training of an AI model (S110). In this case, the dataset may include data obtained by various institutions, data obtained from various equipment, data obtained through various imaging methods, and the like. As a result, there may be a domain difference.

The training apparatus 100 trains an AI model using the dataset while reducing the domain difference in the input data (S120). As a domain generalization method for the above-described process, a method for removing domain features of the input data through pre-processing the input data and a method for removing the domain features from features extracted from the AI model may be used.

The training apparatus 100 may perform pre-processing for removing the domain features of the input data, and then train the AI model using the pre-processed input data. For example, the training apparatus 100 may perform pre-processing of removing the domain features of images obtained from different domains, through image-to-image translation. With a discriminator and an adversarial loss, the training apparatus 100 may train an image-to-image translation model converting the input data so that the discriminator cannot distinguish a domain of images.

Alternatively, with the discriminator, the training apparatus 100 may train the AI model so as not to discern the domain from intermediate features extracted from the middle of the AI model.

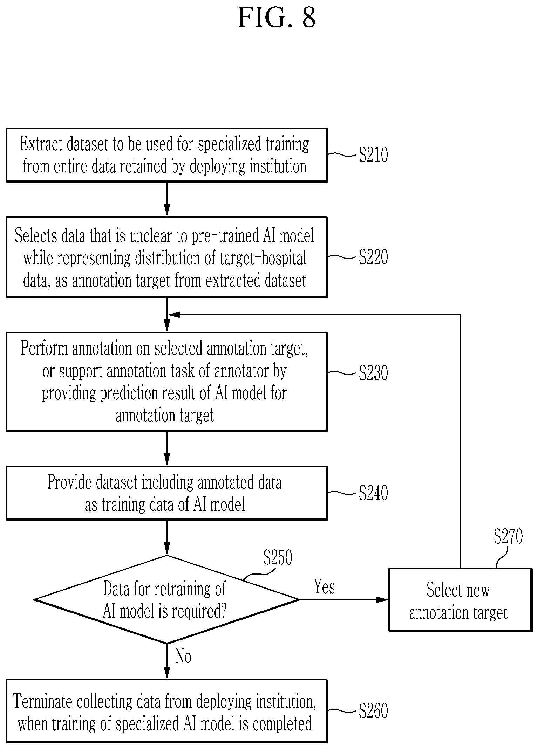

FIG. 8 is a flowchart showing a method for collecting data for specialized training according to an embodiment.

Referring to FIG. 8, the training apparatus 100 extracts a dataset to be used for specialized training from the entire data retained by a deploying institution (e.g., a target hospital) (S210). The dataset used for specialized training may include abnormal training data, normal training data, and validation data, and may be determined according to the number of lesions and a collected number of each lesion. In this case, the training apparatus 100 may determine an amount of data used for training in consideration of data retention amount and data characteristics of the deploying institution. The amount of data used for specialized training may vary in each institution. The data characteristics may include a proportion of abnormal data (data proportion of severe cases), an age distribution, a gender distribution, a racial distribution, and the like.

The training apparatus 100 selects data that is uncertain to a pre-trained AI model while representing a distribution of target-hospital data, as an annotation target from an extracted dataset (S220). The training apparatus 100 may select data uncertain to the pre-trained AI model or data representing the distribution of the target-hospital data. The training apparatus 100 may use uncertainty and/or diversity of the target-hospital data to select the annotation target. The uncertainty may be measured by using a confidence value of a score for each lesion predicted in the pre-trained AI model 200, measured by using entropy of a heatmap for each lesion predicted in the pre-trained AI model 200, or measured in consideration of co-occurrence of the lesions. For diversity representing the distribution of the target-hospital data, the annotation target may be randomly sampled or k representative data may be selected as the annotation target. The annotation target may be selected from data for which a radiologist report exists. An amount of annotation data may be variously determined according to performance of the specialized AI model, or may be determined as a certain amount according to a request from the deploying institution. Meanwhile, as described above using examples with reference to FIG. 3 to FIG. 5, the AI model may be trained without separate annotation on the target-hospital data.

The training apparatus 100 performs annotation on the selected annotation target or supports an annotation task of an annotator by providing a prediction result of the AI model for the annotation target (S230). The training apparatus 100 may annotate information extracted from the radiologist report, as a label of the annotation target. For the annotator, the training apparatus 100 may provide a prediction result of the AI model 200 for the annotation target, and operate so that the annotation task is semi-automatically performed. The training apparatus 100 may provide the annotation target to the annotator and receive a label of the annotation target.

The training apparatus 100 provides a dataset including the annotated data as training data of the AI model (S240).

The training apparatus 100 determines whether data for retraining of the AI model is required according to a validation result of the AI model trained with the training data (S250).

When training of the specialized AI model is completed, the training apparatus 100 terminates collecting data from the deploying institution (S260).

When the data for re-training is required, the training apparatus 100 selects unannotated data as a new annotation target (S270). The training apparatus 100 may select new data that has not been extracted among the entire data retained by the deploying institution.

As described above, the training apparatus 100 iterates reselection of the annotation target or re-extraction of the target-hospital data until training is completed, and provides a new dataset including the annotated data, as data for specialized training of the AI model.

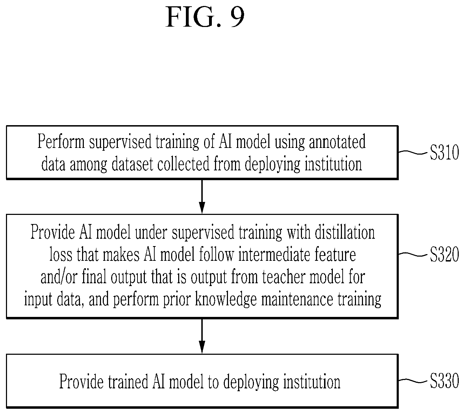

FIG. 9 is a flowchart showing a specialized training method according to an embodiment.

Referring to FIG. 9, a training apparatus 100 trains an AI model so as not to forget prior knowledge obtained through pre-training while training the AI model with data collected from the deploying institution. To this end, a distillation loss that makes an AI model (student model) under specialized training follow an intermediate feature and/or a final output of the pre-trained AI model (teacher model) is used. As a result, the AI model can learn the new data without forgetting the prior knowledge.

The training apparatus 100 performs supervised training of the AI model using annotated data among the dataset collected from the deploying institution (S310). For specialized training, label-annotated target-hospital data may be used and label-annotated prior knowledge data may also be used. The training apparatus 100 may calculate a supervised loss through comparing an output of the AI model for input data and a label, and may backpropagate the supervised loss to the AI model. The training apparatus 100 provides an AI model under supervised training with a distillation loss that makes the AI model follow the intermediate feature and/or final output that is output from the teacher model for the input data, thereby performing prior knowledge maintenance training (S320). The teacher model may be an AI model pre-trained with the pre-training data. An initial model of the AI model under supervised training may be the pre-trained AI model. For prior knowledge maintenance training, the target-hospital data or prior knowledge may be used, and data without labels may also be used. Meanwhile, the training apparatus 100 may select data to be used for prior knowledge maintenance training among the pre-training data or the target-hospital data, by using metrics such as a score for each lesion, an uncertainty score for each lesion, and an abnormality score.

The training apparatus 100 provides the trained AI model to the deploying institution (S330). That is, when the specialized AI model outputs good performance on the target-hospital data, it is provided for the use of the deploying institution.

The training apparatus 100 may simultaneously progress prior knowledge maintenance training and specialized training, by calculating weighted sum of a supervised loss and a distillation loss according to weights and back-propagating the weighted-summed loss to the AI model under training.

Meanwhile, the training apparatus 100 may validate the trained AI model by using validation data collected from the deploying institution. Validation with the validation data may be performed as necessary. According to a validation result, the training apparatus 100 reselects the target-hospital data for retraining, and performs retraining of the AI model using the reselected data.

As above-described, the AI model retains the prior knowledge learned with the pre-training data without forgetting them while learning a task using annotated target-hospital data. Therefore, according to the present disclosure, the AI model can stably function without catastrophic forgetting of losing prior knowledge pre-learned by the target-hospital data while passing through localization to a domain of the deploying institution using the target-hospital data.

On the other hand, the AI model can perform specialized training using the annotated target-hospital data, and can perform specialized training using the unannotated target-hospital data as described with reference to FIG. 3 to FIG. 5.

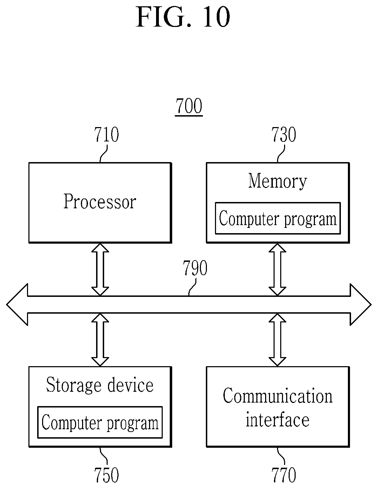

FIG. 10 is a configuration diagram of a computing device according to an embodiment.

Referring to FIG. 10, a training apparatus 100 or a basic training apparatus 110, a data manager 130, and a specialized training apparatus 150 constituting the training apparatus 100 are implemented with a computing device 700 operated by at least one processor.

The computing device 700 includes one or more processors 710, a memory 730 for loading a computer program executed by the processor 710, a storage device 750 for storing the computer program and various data, a communication interface 770, and a bus 790 connecting them. In addition, the computing device 700 may further include various components. The processor 710 is a device that controls operations of the computing device 700, and may be a processor of various types that processes instructions included in the computer program. For example, the processor 710 may be configured to include at least one of a central processing unit (CPU), a micro processor unit (MPU), a micro controller unit (MCU), a graphic processing unit (GPU), or any type of processor well known in the art of the present disclosure.

The memory 730 stores various data, commands, and/or information. The memory 730 may make instructions described to operations of the present disclosure be processed by the processor 710 through loading a corresponding program from the storage device 750. The memory 730 may be, for example, a read only memory (ROM), a random access memory (RAM), and the like.

The storage device 750 may non-temporarily store a computer program and various data. The storage device 750 may be composed to include a hard disk, a removable disk, a non-volatile memory such as a read only memory (ROM), an erasable programmable ROM (EPROM), an electrically erasable programmable ROM (EEPROM), and a flash memory, or any type of computer-readable medium well-known in the art of the present disclosure.

The communication interface 770 may be a wired/wireless communication module supporting wired/wireless communication.

The bus 790 provides communication functions between components of the computing device 700.

The computer program includes instructions executed by the processor 710, and is stored on a non-transitory computer-readable storage medium. The instructions enable the processor to execute the operations of the present disclosure. The computer program may be downloaded via a network or sold as a product.

The computer program according to an embodiment may include instructions for collecting a dataset for pre-training of an AI model and training the AI model using the collected dataset according to a predetermined domain generalization method. The computer program may include instructions for pre-processing to remove domain features of the input data and training the AI model with the pre-processed input data. The computer program may include instructions that pre-processes to remove the domain features of images acquired from different domains through image-to-image translation and trains an image-to-image translation model using a discriminator and an adversarial loss so as to convert the input data in order for a discriminator not to distinguish a domain of an image. The computer program may include instructions for training the AI model so as not to discern the domain from intermediate features extracted from the middle of the AI model through the discriminator.

A computer program according to another embodiment may include instructions for extracting a dataset used for specialized training from all data retained by a deploying institution, instructions for selecting, as an annotation target, uncertain data to a pre-trained AI model while representing a distribution of a target-hospital data from the extracted dataset, instructions for performing annotation on the annotation target or supporting an annotation task, and instructions for providing a dataset including the annotated data as training data of an AI model. Further, the computer program may include instructions for selecting new data not been extracted from the entire data retained by the deploying institution or generating a new dataset through selecting unannotated data as a new annotation target in a case where data for retraining of the AI model is required according to a validation result of the AI model trained with the training data.

A computer program according to another embodiment may include instructions for performing supervised training of an AI model by using annotated data among datasets collected from a deploying institution. The computer program may include instructions for providing an AI model under supervised training with a distillation loss that makes the AI model follow an intermediate feature and/or a final output that is output from the teacher model for the input data to perform prior knowledge maintenance training. The computer program may include instructions for selecting data to be used for prior knowledge maintenance training from the target-hospital data by using metrics such as a prediction score for each lesion, an uncertainty score for each lesion, and an abnormality score. The computer program may include instructions for validating a trained AI model by using validation data collected from a deploying institution, instructions for reselecting target-hospital data for retraining and performing retraining of the AI model using the reselected data in a case where performance validation falls short of standard, and instructions for making a training apparatus 100 terminate training and providing trained AI model to a deploying institution in a case where the performance validation is satisfied.

According to an embodiment, since various institutions can use an AI model specialized for domain characteristics of each institution, there is no need to worry about degradation of the AI model performance due to a difference in domains such as imaging equipment, imaging method, severity, and race.

According to an embodiment, the AI model may learn intrinsic data of each institution while maintaining prior knowledge for stable operation. Therefore, according to an embodiment, an AI model specialized for each institution can provide an analysis result reflecting intrinsic characteristics of each institution while providing generalization performance.

According to an embodiment, since data that is uncertain while representing data retained by each institution may be selected as an annotation target among the data retained by each institution, annotation only on the selected data may be required needless to annotate all data. Therefore, according to an embodiment, convenience in training may be increased, training time may be reduced, and training cost may be saved.

According to an embodiment, a provider providing an AI model to institutions may differentiate the AI model by changing an amount of collected data or an amount of data requiring annotation according to contract terms with each institution.

The embodiment of the present disclosure described above is not implemented with only an apparatus and a method, and may be implemented with a program for executing functions corresponding to a configuration of an embodiment of the present disclosure or a recording medium in which the program is recorded.

While this invention has been described in connection with what is presently considered to be practical embodiments, it is to be understood that the invention is not limited to the disclosed embodiments. On the contrary, it is intended to cover various modifications and equivalent arrangements included within the scope of the appended claims.

## Claims

1.    A method for operating a training apparatus operated by at least one processor, the method comprising:

   extracting a medical institution dataset to be used for specialized training from data retained by a first medical institution;
   selecting an annotation target for which annotation is required, from the medical institution dataset by using a pre-trained artificial intelligence (AI) model previously pretrained on data from a second medical institution different than the first medical institution, wherein selecting the annotation target comprises selecting uncertain data to the pre-trained AI model as the annotation target by using a prediction result of the pre-trained AI model for at least some data in the medical institution dataset; and
   performing specialized training of the pre-trained AI model by using data annotated with a label for the annotation target to generate a specialized AI model for the first medical institution,
   wherein the medical institution dataset comprises medical images, and
   wherein the pre-trained AI model and the specialized AI model receive medical images as input and are trained for detecting at least one lesion in the medical images.

2.    The method of claim 1, wherein selecting the annotation target comprises selecting the annotation target based on an uncertainty score measured by using the prediction result of the pre-trained AI model.

3.    The method of claim 2, wherein the uncertainty score is measured by using at least one of a confidence value of a score for each lesion predicted in the pre-trained AI model, entropy of a heat map for each lesion predicted in the pre-trained AI model, and co-occurrence of lesions predicted in the pre-trained AI model.

4.    A training apparatus comprising:

   a memory configured to store instructions; and
   a processor configured to execute the instructions,

wherein the processor

extracts a medical institution dataset to be used for specialized training from data retained by a first medical institution,

selects an annotation target for which annotation is required, from the medical institution dataset by using a pre-trained artificial intelligence (AI) model previously pretrained on data from a second medical institution different than the first medical institution, wherein selecting the annotation target comprises selecting uncertain data to the pre-trained AI model as the annotation target by using a prediction result of the pre-trained AI model for at least some data in the medical institution dataset; and performs specialized training of the pretrained AI model by using data annotated with a label for the annotation target to generate a specialized AI model for the first medical institution

wherein the medical institution dataset comprises medical images, and

wherein the pre-trained AI model and the specialized AI model receive medical images as input and are trained for detecting at least one lesion in the medical images.

**5.** The training apparatus of claim 4,

wherein the processor makes prior knowledge of the pre-trained AI model maintained by providing the specialized AI model under supervised training with information for maintaining the prior knowledge.

**Patentansprüche**

**1.** Verfahren zum Betreiben einer Trainingsvorrichtung, die von mindestens einem Prozessor betrieben wird, wobei das Verfahren umfasst:

Extrahieren eines Datensatzes einer medizinischen Einrichtung, der für spezialisiertes Training verwendet werden soll, aus Daten, die von einer ersten medizinischen Einrichtung gehalten werden;
Auswählen eines Anmerkungsziels, für das eine Anmerkung erforderlich ist, aus dem Datensatz der medizinischen Einrichtung unter Verwendung eines vortrainierten Künstliche-Intelligenz- (KI-) Modells, das zuvor mit Daten von einer zweiten medizinischen Einrichtung, die sich von der ersten medizinischen Einrichtung unterscheidet, vortrainiert wurde, wobei das Auswählen des Anmerkungsziels das Auswählen von unsicheren Daten für das vortrainierte KI-Modell als Anmerkungsziel unter Verwendung eines Vorhersageergebnisses des vortrainierten KI-Modells für mindestens einige Daten im Datensatz der medizinischen Einrichtung umfasst; und
Durchführen von spezialisiertem Training des vortrainierten KI-Modells unter Verwendung von Daten, die mit einem Etikett für das Anmerkungsziel vermerkt sind, um ein spezialisiertes KI-Modell für die erste medizinische Einrichtung zu generieren,
wobei der Datensatz der medizinischen Einrichtung medizinische Bilder umfasst, und
wobei das vortrainierte KI-Modell und das spezialisierte KI-Modell medizinische Bilder als Eingang empfangen und zum Erkennen von mindestens einer Läsion in den medizinischen Bildern trainiert werden.

**2.** Verfahren nach Anspruch 1, wobei das Auswählen des Anmerkungsziels das Auswählen des Anmerkungsziels auf Basis einer Unsicherheitsbewertung umfasst, die unter Verwendung des Vorhersageergebnisses des vortrainierten KI-Modells gemessen wird.

**3.** Verfahren nach Anspruch 2, wobei die Unsicherheitsbewertung unter Verwendung mindestens eines von einem Konfidenzwert einer Bewertung für jede Läsion, die in dem vortrainierten KI-Modell vorhergesagt wurde, Entropie einer Heatmap für jede Läsion, die in dem vortrainierten KI-Modell vorhergesagt wurde, und gleichzeitigem Auftreten von Läsionen, die in dem vortrainierten KI]-Modell vorhergesagt wurden, gemessen wird.

**4.** Trainingsvorrichtung, umfassend:

einen Speicher, der so konfiguriert ist, dass er Anweisungen speichert; und
einen Prozessor, der so konfiguriert ist, dass er die Anweisungen ausführt,
wobei der Prozessor
aus Daten, die von einer ersten medizinischen Einrichtung gehalten werden, einen Datensatz der medizinischen Einrichtung extrahiert, der für spezialisiertes Training verwendet werden soll,
ein Anmerkungsziel, für das eine Anmerkung erforderlich ist, aus dem Datensatz der medizinischen Einrichtung unter Verwendung eines vortrainierten Künstliche-Intelligenz- (KI-) Modells auswählt, das zuvor mit Daten von einer zweiten medizinischen Einrichtung, die sich von der ersten medizinischen Einrichtung unterscheidet, vortrainiert wurde, wobei das Auswählen des Anmerkungsziels das Auswählen von unsicheren Daten für das vortrainierte KI-Modell als Anmerkungsziel unter Verwendung eines Vorhersageergebnisses des vortrainierten KI-Modells für mindestens einige Daten in dem Datensatz der medizinischen Einrichtung umfasst; und spezialisiertes Training des vortrainierten KI-Modells unter Verwendung von Daten durchführt, die mit einem Etikett für das Anmerkungsziel vermerkt sind, um ein spezialisiertes KI-Modell für die erste medizinische Einrichtung zu erzeugen,

wobei der Datensatz der medizinischen Einrichtung medizinische Bilder umfasst, und

wobei das vortrainierte KI-Modell und das spezialisierte KI-Modell medizinische Bilder als Eingang empfangen und zum Erkennen von mindestens einer Läsion in den medizinischen Bildern trainiert werden.

**5.** Trainingsvorrichtung nach Anspruch 4,

wobei der Prozessor Vorkenntnisse über das vortrainierte KI-Modell, das gepflegt wird, gewinnt, indem er dem spezialisierten KI-Modell unter überwachtem Training Informationen zur Pflege der Vorkenntnisse bereitstellt.

**Revendications**

**1.** Procédé de fonctionnement d'un appareil d'entraînement fonctionnant au moyen d'au moins un processeur, le procédé comprenant :

l'extraction d'un ensemble de données d'institution médicale à utiliser pour un entraînement spécialisé à partir de données conservées par une première institution médicale ;

la sélection d'une cible d'annotation pour laquelle une annotation est requise, à partir de l'ensemble de données d'institution médicale en utilisant un modèle d'intelligence artificielle (IA) pré-entraîné précédemment pré-entraîné sur des données d'une seconde institution médicale différente de la première institution médicale, où la sélection de la cible d'annotation comprend la sélection de données incertaines pour le modèle IA pré-entraîné comme étant la cible d'annotation en utilisant un résultat de prédiction du modèle IA pré-entraîné pour au moins certaines données dans l'ensemble de données d'institution médicale ; et

la réalisation d'un entraînement spécialisé du modèle IA pré-entraîné en utilisant des données annotées avec une étiquette pour la cible d'annotation afin de générer un modèle IA spécialisé pour la première institution médicale,

dans lequel l'ensemble de données d'institution médicale comprend des images médicales, et

dans lequel le modèle IA pré-entraîné et le modèle IA spécialisé reçoivent des images médicales en tant qu'entrée et sont entraînés pour détecter au moins une lésion dans les images médicales.

**2.** Procédé selon la revendication 1, dans lequel la sélection de la cible d'annotation comprend la sélection de la cible d'annotation sur la base d'un score d'incertitude mesuré en utilisant le résultat de prédiction du modèle IA pré-entraîné.

**3.** Procédé selon la revendication 2, dans lequel le score d'incertitude est mesuré en utilisant au moins l'une parmi une valeur de confiance d'un score pour chaque lésion prédite dans le modèle IA pré-entraîné, l'entropie d'une carte thermique pour chaque lésion prédite dans le modèle IA pré-entraîné, et la cooccurrence de lésions prédites dans le modèle IA pré-entraîné.

**4.** Appareil d'entraînement comprenant :

une mémoire configurée pour stocker des instructions ; et

un processeur configuré pour exécuter les instructions,

dans lequel le processeur

extrait un ensemble de données d'institution médicale à utiliser pour un entraînement spécialisé à partir de données conservées par une première institution médicale,

sélectionne une cible d'annotation pour laquelle une annotation est requise, à partir de l'ensemble de données d'institution médicale en utilisant un modèle d'intelligence artificielle (IA) pré-entraîné précédemment pré-entraîné sur des données d'une seconde institution médicale différente de la première institution médicale, où la sélection de la cible d'annotation comprend la sélection de données incertaines pour le modèle IA pré-entraîné comme étant la cible d'annotation en utilisant un résultat de prédiction du modèle IA pré-entraîné pour au moins certaines données dans l'ensemble de données d'institution médicale ; et réalise un entraînement spécialisé du modèle IA pré-entraîné en utilisant des données annotées avec une étiquette pour la cible d'annotation afin de générer un modèle IA spécialisé pour la première institution médicale

dans lequel l'ensemble de données d'institution médicale comprend des images médicales, et

dans lequel le modèle IA pré-entraîné et le modèle IA spécialisé reçoivent des images médicales en tant qu'entrée et sont entraînés pour détecter au moins une lésion dans les images médicales.

5. Appareil d'entraînement selon la revendication 4,
dans lequel le processeur maintient les connaissances préalables du modèle IA pré-entraîné en fournissant au modèle IA spécialisé sous entraînement supervisé des informations pour maintenir les connaissances préalables.

# FIG. 1

FIG. 2

EP 4 036 931 B1

FIG. 3

# FIG. 4

# FIG. 5

EP 4 036 931 B1

# FIG. 6

Basic AI model

Prior Knowledge → Specialized AI model in training

Trained →

Trained Specialized AI model

① Pre-training

⑨ Reselect/reextract

⑦ Specialized training

⑧ Validate

Pre-training Data

Normal training data

Abnormal training data (labeled or unlabeled)

Validation data

⑥ Annotation

Annotation target

⑤ Select

Abnormal training data

③ Extract

② Extract

④ Extract

⑩ Provide

Target hospital

10

Data repository (e.g., PACS)

FIG. 7

Collect dataset for pre-training
of AI model — S110

Train AI model using dataset while reducing
domain difference in input data — S120

# FIG. 8

Extract dataset to be used for specialized training
from entire data retained by deploying institution — S210

Selects data that is unclear to pre-trained AI model
while representing distribution of target-hospital
data, as annotation target from extracted dataset — S220

Perform annotation on selected annotation target,
or support annotation task of annotator by
providing prediction result of AI model for
annotation target — S230

Provide dataset including annotated data
as training data of AI model — S240

S250
Data for retraining of
AI model is required? —— Yes —— Select new
annotation target — S270

No

Terminate collecting data from deploying institution,
when training of specialized AI model is completed — S260

# FIG. 9

Perform supervised training of AI model using annotated data among dataset collected from deploying institution —S310

↓

Provide AI model under supervised training with distillation loss that makes AI model follow intermediate feature and/or final output that is output from teacher model for input data, and perform prior knowledge maintenance training —S320

↓

Provide trained AI model to deploying institution —S330

# FIG. 10

700

710

730

Processor

Memory

Computer program

790

Storage device

Computer program

Communication interface

750

770

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **OZDEMIR FIRAT et al.** *Extending pretrained segmentation networks with additional anatomical structures*